(19) Europäisches Patentamt
European Patent Office
Office européen des brevets

(11) EP 0 517 371 B1

(12) **EUROPEAN PATENT SPECIFICATION**

(45) Date of publication and mention of the grant of the patent:
**10.01.1996 Bulletin 1996/02**

(51) Int. Cl.$^6$: **A61K 7/06**

(21) Application number: **92304042.2**

(22) Date of filing: **05.05.1992**

(54) **Hair growth composition containing citric acid esters**

Zitronensäureester enthaltendes Haarwuchsmittel

Composition pour stimuler la croissance des cheveux contenant des esters de l'acide citrique

(84) Designated Contracting States:
**AT BE CH DE DK ES FR GB GR IT LI NL PT SE**

(30) Priority: **07.05.1991 GB 9109734**

(43) Date of publication of application:
**09.12.1992 Bulletin 1992/50**

(73) Proprietors:
- **UNILEVER PLC**
  **London EC4P 4BQ (GB)**
  Designated Contracting States:
  **GB**
- **UNILEVER N.V.**
  **NL-3013 AL Rotterdam (NL)**
  Designated Contracting States:
  **BE CH DE DK ES FR GR IT LI NL PT SE AT**

(72) Inventors:
- **Raman, Govindrajan**
  **Chakala, Andheri East, Bombay 400 099 (IN)**
- **Natraj, Collur Visweswaria**
  **Chakala, Andheri East, Bombay 400 099 (IN)**

(74) Representative: **Mulder, Cornelis Willem Reinier, Dr. et al**
**Sharnbrook, Bedford MK44 1LQ (NL)**

(56) References cited:
**EP-A- 0 102 534**
**EP-A- 0 188 749**
**EP-A- 0 277 428**
**EP-A- 0 334 585**

- **PATENT ABSTRACTS OF JAPAN vol. 11, no. 365 (C-460)27 November 1987**
- **Aldrich Katalog 90/91 page 377, reference: 10,929-0**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.9.1/3.4

## Description

The invention relates to cosmetic and pharmaceutical compositions for topical application to mammalian skin or hair, containing an ester of citric acid which is capable of promoting hair growth, especially terminal hair growth on the human scalp.

### The Hair Growth Cycle

It should be explained that in most mammals, hair does not grow continuously, but undergoes a cycle of activity involving alternate periods of growth and rest. The hair growth cycle can be divided into three main stages, namely:

(i) the growth phase known as anagen, during which the hair follicle penetrates deep into the dermis with the cells of the bulb dividing rapidly and differentiating to form the hair,

(ii) the transitional stage known as catagen, which is heralded by the cessation of mitosis, and during which the follicle regresses upwards through the dermis and hair growth ceases,

(iii) the resting stage known as telogen, in which the regressed follicle contains a small secondary germ with an underlying ball of tightly packed dermal papilla cells.

The initiation of a new anagen phase is revealed by rapid proliferation in the germ, expansion of the dermal papilla and elaboration of basement membrane components. The hair cycle is then repeated many times until, as a consequence of the onset of male pattern baldness, most of the hair follicles spend an increasing proportion of their time in the telogen stage, and the hairs produced become finer, shorter, and less visible; this is known as terminal to vellus transformation.

### Alleged Baldness Cures

Although there have been many claims in the scientific literature to the promotion or maintenance of hair growth by the topical application of hair tonics and the like, with the possible exception of minoxidil, none has been shown to be sufficiently free from disadvantageous clinical side effects, whether administered topically, orally or systemically, to warrant commercial exploitation as an ethical pharmaceutical, proprietary medicine, or as a cosmetic product. Possibly, the only means which has met with partial success for growing hair on the bald or balding human head is by transplantation of hair to the bald areas. This is, however, an extremely painful operation and is not always successful. Furthermore, it is immediately apparent to the casual observer that the subject has received a hair transplant and it may take many months or even years before hair regrowth, following this operation, assumes an appearance which resembles that of the original naturally growing hair.

Among the many hair regrowth studies that have been reported in the literature, there is included the work of Bazzano as described in PCT International Publication No. WO 85/04577. This publication describes a composition which is useful for increasing the rates of hair growth on mammalian skin, prolonging the anagen phase of the hair growth cycle and for treating various types of alopecias. The composition in question comprises a pyrimidine carbamate.

It has also been reported in US patent no. 4 139 619 to Chidsey assigned to the Upjohn Company, that a topical composition comprising minoxidil as the free base or acid addition salt thereof, or certain specified related iminopyrimidines, is useful in stimulating the conversion of vellus hair to growth as terminal hair, as well as increasing the rate of growth of terminal hair.

In spite of the apparent stimulation of hair growth or regrowth reported independently by Bazzano and Chidsey, following topical application of minoxidil or related compounds, there is general concern that systemic side-effects can result, particularly following topical application of minoxidil. Thus it is generally recognised in the medical literature that the side effects of orally administered minoxidil are very serious, and include fluid retention, tachycardia, dyspnea, gynecomastia, fatigue, nausea and cardiotoxicity. There is also evidence that certain side effects have been experienced following topical application of minoxidil.

As a result of a programme of screening substances for their ability to treat or prevent photo-damage of skin following excessive exposure to sunlight, we discovered that certain esters of citric acid, especially tributyl citrate were particularly effective in this respect. Thus, certain unexpected responses have been observed which suggested that these substances may also be capable of promoting hair growth. This was tested and evidence obtained to substantiate this hypothesis.

The invention is accordingly concerned with compositions comprising certain esters of citric acid, optionally in combination with other hair growth promoters and/or penetration enhancers for topical application to human skin, particularly the scalp, in order to promote hair growth and/or to reduce natural hair loss, to an extent which is entirely unexpected and indeed which is moreover synergistic.

Accordingly, in one aspect, the invention provides the use of a preserved composition, which comprises:

an effective amount of from 1% to 99% by weight of an ester of citric acid having the structure (1):

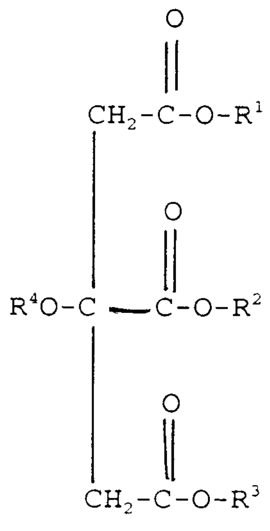

(1)

where

$R^1$, $R^2$ and $R^3$ each independently represent branched or unbranched alkyl, alkenyl, aryl, alkylaryl or arylalkyl group each said group having from 1 to 18 carbon atoms,

$R^4$ represents -H, or a branched or unbranched saturated or unsaturated acyl, alkyl, aryl, alkylaryl or arylalkyl group having from 1 to 18 carbon atoms,

in the presence of a cosmetically acceptable vehicle for the citric acid ester and in the absence of solid absorbent for the ester; for inducing, maintaining or increasing hair growth following topical application to human scalp or hair.

In another aspect, the invention provides for use of an ester of citric acid having the aforementioned structure (1) of inducing, maintaining or increasing hair growth following topical application to human scalp or hair.

<u>The ester of citric acid</u>

The composition according to the invention comprises an ester of citric acid or mixtures thereof, each having the structure (1).

The preferred esters of citric acid are those where the $R^1$, $R^2$ and $R^3$ groups in structure (1) are each alkyl groups, examples of which include:

trimethyl citrate
triethyl citrate
tri-n-propyl citrate
tri-n-butyl citrate
trihexyl citrate
trioctyl citrate
tridodecyl citrate
trihexadecyl citrate
triphenyl citrate
tri-2-ethylhexyl citrate.

Typically $R^1$, $R^2$ and $R^3$ are alkyl groups, preferably unsubstituted, having from 1 to 12 carbon atoms, particularly 1 to 8, most preferably 4 to 6 carbon atoms. It is also preferred that $R^1=R^2=R^3$.

The above preferred trialkyl citrates can also be acylated at the 2 position, further to enhance their liophilic character, and examples of these are:

2-acetyl trimethyl citrate
2-acetyl triethyl citrate
2-acetyl tri-n-propyl citrate
2-acetyl tri-n-butyl citrate
2-oleoyl tri-n-butyl citrate
2-0-methyl tri-n-butyl citrate

2-0-ethyl tri-n-butyl citrate

2-0-isopropyl tri-n-butyl citrate.

The preferred ester of citric acid is tri-n-butyl citrate.

The effective amount which is sufficient to induce, maintain or increase hair growth will depend on the effectiveness of the ester, some being more effective than others, but in general, an amount of from 0.0001 to 99%, preferably from 0.01 to 20% and particularly more than 1% by weight of the composition will provide an adequate dose to the skin topical application.

Preservation of the Composition

The composition according to the invention is preferably preserved in such a manner that it will enjoy an extended shelf life following manufacture and prior to sale and use. Ideally the composition will have an indefinite shelf life.

It is accordingly apparent that the ester is likely to be prone to attack by bacteria, moulds and fungi and other microbial influences, particularly at pH values near that of the skin that characterise the preferred composition. The shelf-life of the composition can therefore be unacceptably short due to the biodegradation of the ester unless steps are taken to preserve the composition.

In order to be preserved, the composition should preferably be free, or substantially free, from viable microbial contaminants that are capable of resulting in microbial spoilage of the composition, and/or biodegradation of the ester prior to topical application of the composition to mammalian skin or hair. It is to be understood, however, that the invention is also concerned with compositions, as herein defined, which may contain viable but dormant microorganisms, such as bacterial spores, provided that the conditions of preservation do not result in substantial proliferation of the microorganisms prior to use of the composition.

Examples of methods that can be employed to achieve preservation of the composition, includes the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to the invention to reduce the water activity ($\alpha_w$) from 1 to < 0.9, preferably to < 0.85 and most preferably < 0.8, the lowest of these values being that at which yeasts, moulds and fungi will not proliferate.

pH

The esters of citric acid may be susceptable to hydrolysis, particularly when the pH value of the composition is alkaline. It is accordingly preferred that the composition, when aqueous, should have an acid pH value. The preferred pH value of the composition, when aqueous, is from 2 to <7, ideally from 4 to 6.5.

The Cosmetically Acceptable Vehicle

The composition according to the invention also comprises a solid, semi-solid or liquid cosmetically and/or physiologically acceptable vehicle, to enable the ester to be conveyed to the scalp at an appropriate dilution, and so long as the vehicle does not absorb the ester to an extent which prevents it penetrating the scalp. The nature of the vehicle will

depend upon the method chosen for topical administration of the composition. The vehicle can itself be inert or it can possess physiological or pharmaceutical benefits of its own.

The selection of a vehicle for this purpose presents a wide range of possibilities depending on the required product form of the composition. Suitable vehicles can be classified as described hereinafter.

It should be explained that vehicles are substances which can act as diluents, dispersants, or solvents for the ester which therefore ensure that they can be applied to and distributed evenly over the hair and/or scalp at an appropriate concentration. The vehicle is preferably one which can aid penetration of the esters into the scalp to reach the immediate environment of the hair follicle. Compositions according to this invention can include water as a vehicle, and/or at least one cosmetically acceptable vehicle other than water.

Vehicles other than water that can be used in compositions according to the invention can include solids or liquids such as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polythylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate; may be acceptable provided they do not absorb the ester and thereby prevent it penetrating the scalp.

The amount of vehicle in the composition, including water if present, should preferably be sufficient to carry at least a portion of a selected ester to the skin in an amount which is sufficient effectively to enhance hair growth. The amount of the vehicle can comprise the balance of the composition, particularly where little or no other ingredients are present in the composition. Accordingly, the vehicle or vehicles can comprise from 1 to 99.99%, preferably from 50 to 99.5% and ideally from 90 to 99% by weight of the composition.

Perfume

The composition according to the invention can also optionally comprise a perfume in an amount sufficient to make the composition acceptable to the consumer and pleasant to use. Usually, the perfume will form from 0.01 to 10% by weight of the composition.

Activity Enhancer

The composition according to the invention can also optionally comprise an activity enhancer.

The activity enhancer can be chosen from a wide variety of molecules which can function in different ways to enhance synergistically the hair growth effects of the ester. Particular classes of activity enhancers include other hair growth stimulants, penetration enhancers and cationic polymers, whose presence can further improve synergistically the delivery of the ester through the stratum corneum to its site of action in the immediate environment of the hair follicle.

Some activity enhancers can also function as vehicles for the ester.

(a) Other Hair Growth Stimulants

i) Examples of other substances which themselves possess the ability to stimulate or increase hair growth include, for example;

Benzalkonium chloride
Benzethonium chloride
Phenol
Estradiol

Diphenhydramine hydrochloride
Chlorpheniramine maleate
Chlorophyllin derivatives
Cholesterol
Salicylic acid
Cystine
Red pepper tincture
Benzyl nicotinate
dl-Menthol
Peppermint oil
Calcium pantothenate
Panthenol
Castor oil
Hinokitiol
Prednisolone
Resorcinol

Further substances which themselves possess the ability to increase the rate of terminal hair growth include:

ii) α-1,4 esterified disaccharides described by Choay S.A. in EP-A-O 064 012, having the structure (2):

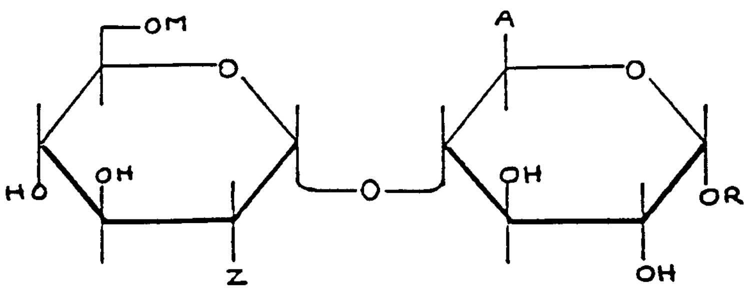

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ to $C_4$ alkyl radical, especially methyl; or an aryl radical;

A represents a functional group such as an acid or $-COOR_1$, where $R_1$ represents -H or a $C_1$ to $C_4$ alkyl radical, especially methyl; or a metal, especially an alkali metal;

iii) esterified oligosaccharides as described by Unilever in EP-A-O 211 610, including at least one esterified disaccharide unit consisting of a uronic acid residue having the structure (3):

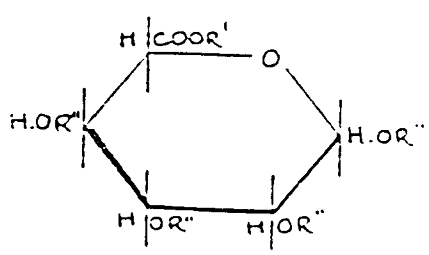

(3)

and a hexosamine residue having the structure (4):

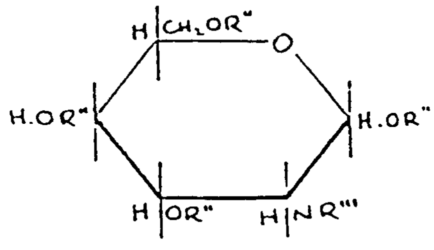

(4)

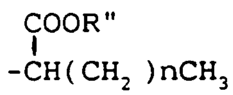

where

R′ is -H, $C_3$ to $C_{10}$ alkyl or

R″ is -H, $C_1$ to $C_4$ alkyl, $-CO(CH_2)_mCH_3$, $-SO_3M$,

R‴ is -H, $-CO(CH_2)mCH_3$, or $-SO_3M$,

M is -H, or a metallic or organic cation

n is 0 or an integer of from 1 to 7, and

m is 0 or the integer 1 or 2;

the groups designated R″ being the same or different, one R″ group from each pyranose ring structure being linked by a glycosidic linkage having the configuration α-1,3, α-1,4, β-1,3 or β-1,4; and the -COOR′, $-CH_2OR''$ and -OR″ groups being of either configuration with respect to the pyranose rings;

iv) Minoxidil glucuronides, as described by Unilever in EP-O 242 967, and Minoxidil sulphates, as described by The Upjohn Co. in WO 86/04231, and

v) Minoxidil, and other derivatives thereof as described by The Upjohn Co, in US patent 4 139 619.

Particularly preferred mixtures of minoxidil and an ester according to the invention include the following:

Minoxidil and tri-n-butyl citrate
Minoxidil and 2-acetyl tri-n-butyl citrate
Minoxidil and tri-n-propyl citrate
Minoxidil and 2-O-ethyl tri-n-butyl citrate.

vi. Ethylenediaminetetraacetic acid or salts thereof, as described by Redken Laboratories, Inc. in US 4 814 351.

vii. Direct proteoglycanase inhibitors, such as 1,10-phenanthroline, as described by Unilever in EP-0 277 428.

viii.Glycosaminoglycanase inhibitors, as described by Unilever in EP-0 277 428, such as aldonolactones and esterified aldonolactones having the structure (33):

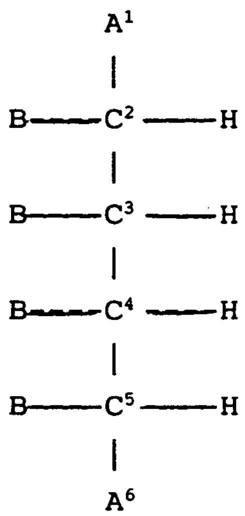

(33)

where

$A^1$ and $A^2$ are -H, $-CH_3$,

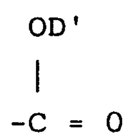

or

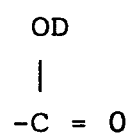

B is OD″ or a lactone linkage to position 1 or 6, or $-NHCOCH_3$

and where

D is -H or $C_2$ to $C_8$ alkyl,

D′ is the remainder of the molecule joined through another C atom at positions 2 to 5 to form a lactone,

D″ is -H or $C_2$ (ie acetyl) to $C_4$ acyl of either configuration with respect to the backbone of this molecule;

preferred examples of which include:

L-Galactono-1,4-lactone

L-Arabino-1,5-lactone

D-Fucono-1,5-lactone

D-Glucaro-1,4-lactone

D-Glucurono-6,3-lactone

Galactaric acid lactone

2-Acetamido-2-deoxygluconolactone

2-Acetamido-2-deoxygalactono-lactone

D-Glucaro-1,4:6,3-dilactone

L-Idaro-1,4-lactone

2,3,5-Tri-0-acetyl-D-glucaro-1,4-lactone

2,5-Di-0-acetyl-D-glucaro-1,4:6,3-dilactone.

ix. Glycosaminoglycanase inhibitors, as described by Unilever in EP 0 277 428, such as monosaccharides and esterified monosaccharides having the structure (34):

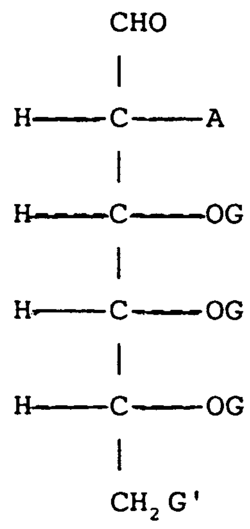

(34)

where
A is -OG or $-NHCOCH_3$
G is -H, $-SO_3M''$, $C_2$ to $C_4$ acyl
G′ is -H or -OG
M″ is -H or a metal cation

wherein the functional groups can be in either configuration with respect to the backbone of the above molecule; preferred examples of which include:

N-Acetylglucosamine
N-Acetylgalactosamine
D-Galactosamine
D-Glucosamine-3-sulphate
N-Acetylmannosamine.

x. Glycosaminoglycan chain cellular uptake inhibitors, as described by Unilever in EP 0 277 428, such as hexuronic acid and esters thereof which may be represented by the generic structure (35):

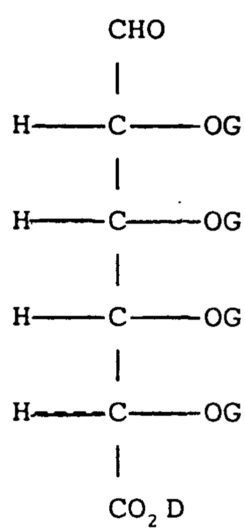

(35)

where
G is -H, $-SO_3M''$, $C_2$ to $C_4$ acyl;
D is -H or $C_2$ to $C_8$ alkyl

M'' is -H or a metal cation;

wherein the functional groups can be in either configuration with respect to the backbone of the above molecule;

xi. Chemical inhibitors of glycosidase activity, as described by Unilever in EP 0 334 586, chosen from lactams having the structure (36):

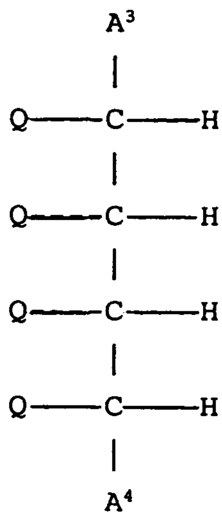

(36)

where

A³ and A⁴ are -H, $-CH_3$,

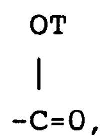

$-CH_2$ OT or

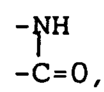

A³ and A⁴ being the same or different, and at least one of which being the group:

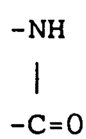

in a lactam ring;

and where Q is -OT', -NHT' or a lactam linkage to A³ or A⁴ ;

the Q groups being the same or different, and at least one of which is involved in a lactam linkage;

and where

T is the same or different and is chosen from -H, $-C_pH_{2p+1}$ or a metal ion,

T′ is -H or

$$-COC_{p}H_{2,p+1},$$

and

p is an integer of from 1 to 22;

provided that:

where any of the Q groups is

-OT′ or -NHT′,

then that group or groups can be of either stereochemical configuration with respect to the plane of the ring, preferred examples of which include:

D-glucaro-1,5-lactam,
L-Galactono-1,4-lactam,
L-Arabino-1,5-lactam,
D-Fucono-1,5-lactam,
D-Glucaro-1,4-lactam,
D-Glucurono-6,3-lactam,
1,2,5-tri-O-acetyl-D-glucurono-6,3-lactam,
2-Acetamido-2-deoxygluconolactam,
2-Acetamido-2-deoxygalactonolactam,
D-Glucaro-1,4:6,3-dilactam,
L-Idaro-1,4-lactam,
2,3,5-Tri-O-acetyl-D-glucaro-1,4-lactam,
2,5-Di-O-acetyl-D-Glucaro-1,4:6,3-dilactam,
D-glucaro-1,5-lactam ethyl ester;

xii. Chemical activators of protein kinase C enzymes, as described by Unilever in EP 0 334 585 chosen from diacylglycerols having the structure (37):

$H_2$ -C-OH
|
H -C-OX
|
$H_2$ -C-OX'

(37)

where

X and X′ are the same or different and is represented by the grouping:

$$-\overset{O}{\overset{||}{C}}-[(CH_2)_{a'}\ (CH{=}CH)_b]\ CH_3$$

where

a is O or an integer of from 1 to 28, and b is 0 or an integer of from 1 to 5;

the X and X′ groups being of either stereochemical configuration with respect to the carbon backbone of the glycerol molecule;

preferred examples of which include:

1,2-Dibutanoyl-rac-glycerol
1,2-Dihexanoyl-sn-glycerol
1,2-Dioctanoyl-rac-glycerol
1,2-Dioctanoyl-sn-glycerol
1,2-Didecanoyl-rac-glycerol
1-Oleoyl-2-acetyl-rac-glycerol
1-Oleoyl-2-acetyl-sn-glycerol
1-Stearoyl-2-arachidonoyl-sn-glycerol
1,2-Distearoyl-rac-glycerol
1,2-Dipentadecanoyl-sn-glycerol
1,2-dipentadecanoyl-rac-glycerol
1,2-Dipalmitoyl-rac-glycerol
1,2-Dipalmitoyl-sn-glycerol
1,2-Diseptadecanoyl-rac-glycerol
1,2-Dioleoyl-sn-glycerol
1,2-Dioleoyl-rac-glycerol
1,2-Diarachidonoyl-sn-glycerol
1,2-Dieicosanoyl-sn-glycerol
1,2-Didoeicosanoyl-rac-glycerol, and
1,2-Dioctaeicosanoyl-sn-glycerol.

xiii.Glycosaminoglycanase inhibitors, as described by Unilever in EP 0 348 184, chosen from aldonomonolactone or alduronomonolactone derivatives having the structure (38):

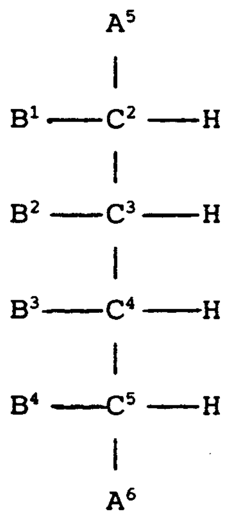

(38)

where
$A^5$ is

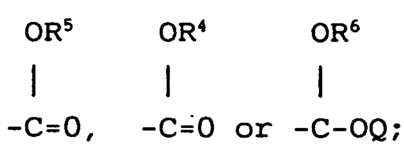

$A^6$ is

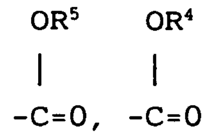

or $CH_2 OR^6$

$B^1$, $B^2$, $B^3$ and $B^4$ are each chosen from is $OR^5$, $NHR^6$, $NHR^7$ or a lactone linkage to position 1 or 6,and/or an ether linkage to $Q^1$;

said substituents B being the same or different, and being in either configuration, with respect to the backbone of the above structure, on positions C2 to C5 not involved in a lactone ring;

and where $R^4$ is -H, $C_1$ to $C_{20}$ alkyl, a metal cation, $NH_4$ +

or an alkanolamine cation;

$R^5$ is the remainder of the molecule joined through another C atom at positions 2 to 5 to form a lactone;

$R^6$ is -H, $-CH_3$, benzyl or $C_2$ to $C_6$ acyl; $R^7$ is -H, $-CH_3$, benzyl or $C_3$ to $C_6$ acyl;

Q1 is the remainder of the molecule joined through an ether linkage to either $C^4$ or $C^5$, forming either a pyranose or furanose ring;

provided that, when $A^5$ is

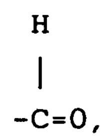

then $A^6$ is

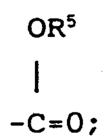

provided also that, when $A^6$ is $CH_2$ OH, then one or more of the B substituents is $-CH_3$, $C_2$ to $C_4$ acyl or $NHR^7$;

provided also that, when $A^5$ is

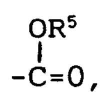

and all $B^1$, $B^2$ $B^3$ and $B^4$ substituents are -OH, then

$A^6$ is

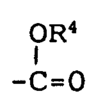

or $CH_2OR^6$, and $R^4$ is $C_1$ or $C_9$ to $C_{20}$ alkyl; preferred examples of which aldonomonolactone derivatives include:

6-acetyl-galactono-1,4-lactone

6-propionyl-galactono-1,4-lactone

6-butyryl-galactono-1,4-lactone

2-propionamido-2-deoxygluconolactone

2-butyramido-2-deoxygluconolactone
2-propionamido-2-deoxygalactonolactone
2-butyramido-2-deoxygalactonolactone
6-propionyl-2-acetamido-2-deoxygluconolactone diacetyl-6-propionyl-2-acetamido-2-deoxygluconolactone
6-butyryl-2-acetamido-2-deoxygalactonolactone diacetyl-6-butyryl-2-acetamido-2-deoxygalactonolactone
2,3,5,6-tetraacetyl-galactono-1,4-lactone
2,3,5-triacetyl-6-propionylgalactono-1,4-lactone
triacetyl-2-propionamido-2-deoxygalactonolactone
triacetyl-2-butyramido-2-deoxygluconolactone
6-methyl-glucaro-1,4-lactone
2,3,5,6-tetramethyl-glucaro-1,4-lactone
6-methyl-2,3,5-triacetylglucaro-1,4-lactone
6-methyl-3-methyl-glucaro-1,4-lactone, and
6-methyl-3-acetyl-glucaro-1,4-lactone;
and a preferred example of which alduronomonolactone derivative is:
1,2,5-triacetyl-glucurono-6,3-lactone.

xiv. Glycosaminoglycanase inhibitors, as described by Unilever in EP 0 348 184, chosen from acylated monosaccharides having the structure (39):

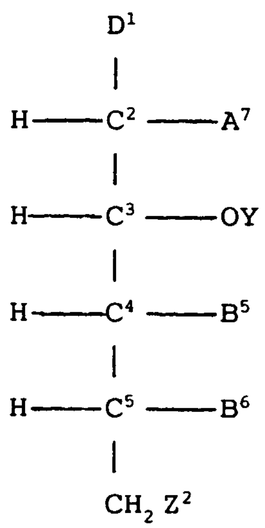

(39)

where
$A^7$ is -OY or -$NHR^8$
$B^5$ and $B^6$ are each chosen from is -OY, or an ether linkage to $D^1$,
$D^1$ is

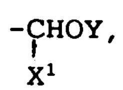

where $X^2$ is an ether linkage either to $C^4$ or $C^5$ forming a pyranose or furanose ring;
Y is -H, -$SO_3M$, $C_2$ to $C_4$ acyl or $C_1$ to $C_{18}$ alkyl;
said substituents $A^7$, $B^5$, $B^6$ and -OY being the same or different, and being in either configuration, with respect to backbone of the above structure;

and where
$Z^1$ is -H or -OY
$R^8$ is -H, -$SO_3M^2$ or $C_3$ or $C_4$ acyl,
$M^2$ is -H, a metal cation, $NH_4$+, or
an alkanolamine cation;
provided that, when $R^8$ is -H, then 1 or more of Y is chosen from -$SO_3M^2$ or $C_2$ to $C_4$ acyl;

and mixtures thereof.

Preferred examples of which acylated monosaccharides include:

2-propionamido-2-deoxyglucose
1,3,4,6-tetraacetyl-2-propionamido-2-deoxyglucose
2-butyramido-2-deoxygalactose
1,3,4,6-tetraacetyl-2-butyramido-2-deoxygalactose
2-sulphamido-2-deoxygalactose
2-sulphamido-2-deoxyglucose
2-butyramido-2-deoxymannose
1,3,4,6-tetraacetyl-2-butyramido-2-deoxymannose
2-butyramido-2-deoxyglucose, and
1,3,4,6-tetraacetyl-2-butyramido-2-deoxyglucose.

xv. Esters of pyroglutamic acid, as described by Lever Brothers Company in US patent No. 4 774 255, having the structure (40):

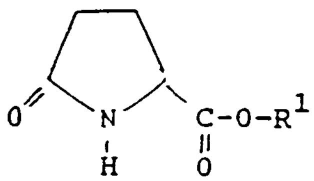

(40)

where

$R^1$ is $C_1$ to $C_{30}$ alkyl, or

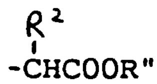

and where $R^2$ and $R^3$ are the same or different and are each represented by H or the grouping (41):

$$[(CH_3)_{u'} (CH_2 OH)_{v'} (CH_2)_{w'}(CH_3 H_2)_{x'} (CHOH)_{y'},(CH=CH)_{z}]- \qquad (41)$$

where

u is zero or 1
v is zero, or the integer 1 or 2,
w is zero, or an integer of from 1 to 21
x is zero, or an integer of from 1 to 4,
y is zero, or the integer 1 or 2,
z is zero, or an integer of from 1 to 4, and u + v + w + x + y + z is an integer of from 1 to 22;

provided that when the subgrouping (CH=CH) is present, then the total number of carbon atoms in said grouping is from 10 to 22.

Examples of suitable esters of pyroglutamic acid where R1 in structure (40) is $C_1$ to $C_{30}$ alkyl are:

pyroglutamic acid methyl ester
pyroglutamic acid ethyl ester
pyroglutamic acid n-propyl ester
pyroglutamic acid n-butyl ester
pyroglutamic acid n-hexyl ester
pyroglutamic acid n-heptyl ester
pyroglutamic acid n-octyl ester
pyroglutamic acid n-nonyl ester
pyroglutamic acid n-decyl ester
pyroglutamic acid n-undecyl ester
pyroglutamic acid n-dodecyl ester
pyroglutamic acid n-tridecyl ester
pyroglutamic acid n-tetradcyl ester
pyroglutamic acid n-hexadecyl ester

pyroglutamic acid n-octadecyl ester
pyroglutamic acid n-eicosyl ester
pyroglutamic acid iso-propyl ester
pyroglutamic acid 2-methylhexyl ester
pyroglutamic acid 2-ethylhexyl ester
pyroglutamic acid 3,7-dimethyloctyl ester
pyroglutamic acid 2-hexyldecyl ester
pyroglutamic acid 2-octyldodecyl ester
pyroglutamic acid 2,4,4-trimetyl-1-pentane ester
pyroglutamic acid methyloctyl ester

Particularly preferred esters of this group are those where R1 in structure (40) is $C_1$ to $C_{14}$ alkyl, (linear or branched); especially $C_1$ to $C_6$ (linear or branched).

Further examples of preferred esters of pyroglutamic acid, where R1 in structure (40) is

$$\begin{array}{c} R^2 \\ | \\ -CHCOOR^3 \end{array},$$

are those where $R^2$ and/or $R^3$ having the structure shown for grouping (41), include straight and branched chain, saturated or unsaturated aliphatic groups having from 1 to 22 carbon atoms, such as the alkyl groups:

methyl
ethyl
propyl
iso-propyl
butyl
iso-butyl
n-valeryl
iso-valeryl
n-caproyl
n-heptyl
n-caprylyl
n-capryl
lauryl
myristyl
palmityl
stearyl, and
arachidyl.

and the $C_{10-22}$ alkenyl groups:

linoleyl
linolenyl
γ-linolenyl
arachidonyl, and
columbinyl.

Further examples of the grouping (24) also include hydroxyalkyl groups having from 1 to 22 carbon atoms, such as:

hydroxymethyl
2-hydroxyethyl
2-hydroxy-n-propyl
3-hydroxy-n-propyl
2-hydroxy-n-butyl
3-hydroxy-n-butyl
4-hydroxy-n-butyl
5-hydroxy-n-valeryl
6-hydroxy-n-caproyl
2,3-dihydroxy-n-propyl

2,3-dihydroxy-n-butyl
12-hydroxystearyl.

Further specific examples of esters of pyroglutamic acid which are particularly suited for use as other hair growth stimulants are:

2-[pyroglutamoyloxy]-propionic acid
methyl-2-[pyroglutamoyloxy]-acetate
ethyl-2-[pyroglutamoyloxy]-n-propionate
ethyl-2-[pyroglutamoyloxy]-n-butyrate
ethyl-2-[pyroglutamoyloxy]-iso-butyrate
ethyl-2-[pyroglutamoyloxy]-n-valerate
ethyl-2-[pyroglutamoyloxy]-n-caproate
ethyl-2-[pyroglutamoyloxy]-n-heptylate
ethyl-2-[pyroglutamoyloxy]-n-caprylate
ethyl-2-[pyroglutamoyloxy]-n-pelargonate
ethyl-2-[pyroglutamoyloxy]-3-hydroxybutyrate
iso-propyl-2-[pyroglutamoyloxy]-n-propionate
iso-propyl-2-[pyroglutamoyloxy]-n-caprylate
n-propyl-2-[pyroglutamoyloxy]-n-propionate
n-propyl-2-[pyroglutamoyloxy]-n-caprylate
stearyl-2-[pyroglutamoyloxy]-n-propionate
12-hydroxystearyl-2-[pyroglutamoyloxy]-n-propionate
stearyl-2-[pyroglutamoyloxy]-n-stearate
palmityl-2-[pyroglutamoyloxy]-n-propionate
linoleyl-2-[pyroglutamoyloxy]-n-propionate
linoleyl-2-[pyroglutamoyloxy]-n-caprylate
lauryl-2-[pyroglutamoyloxy]-n-caprylate
stearyl-2-[pyroglutamoyloxy]-n-caprylate
glyceryl mono(2-[pyroglutamoyloxy]-n-propionate)
glyceryl mono(2-[pyroglutamoyloxy]-n-caprylate), and
glyceryl di(2-[pyroglutamoyloxy]-n-propionate).

xvi. hexosaccharic acids or an acylated hexosaccharic acids, or salts or esters thereof, having the structure (42):

$$\left[\begin{array}{l} COO \\ CHOY^1 \\ CHOY^2 \\ CHOY^3 \\ CHOY^4 \\ COO \end{array}\right]_1 \quad X^1{}_n X^2{}_m \qquad (42)$$

where

$X^1$ is chosen from H, alkalimetal, ammonium and substituted ammonium counterions;
$X^2$ is chosen from an alkyl or hydroxyalkyl group having from 1 to 18 carbon atoms;
$Y^1$, $Y^2$, $Y^3$ and $Y^4$ are each chosen from H, an alkyl group having from 1 to 12 carbon atoms, and an acyl group having from 1 to 18 carbon atoms;
1 is an integer of from 1 to 3;
m and n are each 0 or the integer 1 or 2; and
m+n is 1 or 2.

Examples of hexosaccharic acids, in which $X^1$, $Y^1$, $Y^2$, $Y^3$ and $Y^4$ in the above structure are -H, n is 2, and m is 0, include:

Allosaccharic acid
Altrosaccharic acid
Glucosaccharic acid
Mannosaccharic acid

Gulosaccharic acid
Idosaccharic acid
Galactosaccharic acid, and
Talosaccharic acid.

Examples where $X^1$ is a cation, are the monovalent alkali metal cations $Na^+$ and $K^+$.

Further examples where $X^1$ is a cation are substituted ammonium cations, such as diethanolammonium and triethanolammonium cations.

Examples where $X^2$ is an alkyl group are methyl, ethyl, n-propyl, n-butyl, n-octyl and lauryl.

Examples where $Y^1$, $Y^2$, $Y^3$ and $Y^4$ are alkyl groups, are methyl and ethyl.

Examples where $Y^1$, $Y^2$, $Y^3$ and $Y^4$ are acyl group, are acetyl and propionyl.

A particularly preferred hexosaccharic acid is glucosaccharic acid (also known as saccharic acid or glucaric acid, and hereinafter referred to as glucaric acid) having the structure (43):

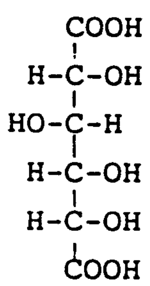

(43)

A particularly stable salt of glucaric acid which is preferred, is the disodium salt.

xvii.aryl-substituted ethylenes having the structure (44)

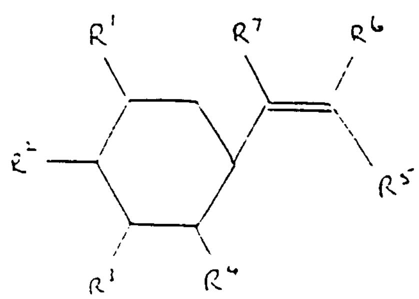

(44)

where $R^1$, $R^2$, $R^3$, & $R^4$ are the same or different, and are chosen from

-H, -OH, $-C_nH_{2n+1}$, $-NO_2$, -Cl, -Br, -F

and

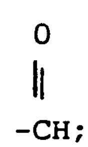

and where

$R^5$ & $R^6$ are the same or different, and are chosen from:

-H, -CN,

```
  O      O          S
  ||     ||         ||
-COH,  -CNH2  and  -CNH2 ;
```

and where $R^7$ is chosen from -H and -OH and where n is an integer of from 1 to 8.

The composition according to the invention can also comprise mixtures of said inhibitors.

Examples of the aryl-substituted ethylenes include
1-carboxy-2-(4-hydroxyphenyl)ethylene
1,1-dicarboxy-2-(4-hydroxyphenyl)ethylene
1,1-dicyano-2-(4-hydroxyphenyl)ethylene
1-carboxy-2-(3,4-dihydroxyphenyl)ethylene
1,1-dicyano-2-(3-hydroxyphenyl)ethylene
1-cyano-1-carboxy-2-(2,5-dihydroxyphenyl)ethylene
1-carboxy-1-cyano-2-(3,4-dihydroxphenyl)ethylene
1,1-dicyano-2-(3,4-dihydroxyphenyl)ethylene
1,1-dicyano-2-(3-methoxy-4,5-dihydroxyphenyl)ethylene
1,1-dicyano-2-(3,4,5-trihydroxyphenyl)ethylene
1-amido-1-cyano-2-(3,4-dihydroxyphenyl)ethylene
1-thioamido-1-cyano-2-(3,4-dihydroxyphenyl)ethylene
1-cyano-2-(4-hydroxyphenyl)ethylene
1,1-dicyano-2-(3-hydroxy-4-nitrophenyl)ethylene
1,1-dicyano-2-hydroxy-2-(4-hydroxyphenyl)ethylene
1,1-dicyano-2-(3-methoxy-4-hydroxyphenyl)ethylene
1,1-dicyano-2-(3,5-dihydroxyphenyl)ethylene
1,1-dicyano-2-hydroxy-2-(3,4,5-trihydroxyphenyl)ethylene
1-carboxy-1-cyano-2-(4-methoxyphenyl)ethylene
1-carboxy-1-cyano-2-(4-fluorophenyl)ethylene
1-carboxy-1-cyano-2-(3-methoxy-4-hydroxyphenyl)ethylene
1-carboxy-1-cyano-2-(3,5-dimethoxy-4-hydroxyphenyl)ethylene
1-carboxy-1-cyano-2-(4-hydroxyphenyl)ethylene
1-carboxy-1-cyano-2-(4-phenylcarboxyaldehyde)ethylene
1-cyano-1-carboxy-2-(2,5-dihydroxyphenyl)ethylene

xviii. mono N-acylated amino acids, in which the acyl group has from 2 to 20 carbon atoms. Examples of which include:

N-acetyl glycine
N-acetyl hydroxyproline
N-acetyl alanine
N-acetyl valine
N-acetyl leucine
N-acetyl isoleucine
N-acetyl phenylalanine
N-acetyl tyrosine
N-acetyl proline
N-acetyl serine
N-acetyl threonine
N-acetyl cysteine
N-acetyl methionine
N-acetyl tryptophan
N-lauroyl glycine
N-palmitoyl glycine
N-myristoyl glycine
N-lauroyl hydroxyproline
N-octanoyl glycine

N-octanoyl hydroxyproline
N-hexanoyl glycine
N-acetyl aspartic acid
N-lauroyl aspartic acid
N-palmitoyl aspartic acid
N-octanoyl aspartic acid
N-acetyl glutamic acid
N-lauroyl glutamic acid
N-palmitoyl glutamic acid
N-octanoyl glutamic acid
N-acetyl arginine
N-acetyl lysine
N-acetyl histidine
N-acetyl ornithine
N-acetyl hydroxylysine
N-acetyl citrulline
N-lauroyl lysine
N-lauroyl citrulline
N-myristoyl citrulline
N-myristoyl ornithine
N-octanoyl lysine, and
N-octanoyl citrulline

xix. Saturated or unsaturated aliphatic alcohols having an odd number of carbon atoms of from 3 to 25 in number, examples of which include:

n-propionyl alcohol
n-amyl alcohol
n-heptyl alcohol
n-nonyl alcohol
n-undecyl alcohol
n-tridecyl alcohol
n-pentadecyl alcohol
n-heptadecyl alcohol
n-nonadecyl alcohol
n-uneicosyl alcohol
n-tricosyl alcohol
n-pentacosyl alcohol

Preferred alcohols, as defined above, are those having from 7 to 15 carbon atoms in number.

xx. Saturated or unsaturated aliphatic carboxylic acids having an odd number of carbon atoms of from 3 to 25 in number, examples of which include:

propionic acid
valeric acid
heptanoic acid
nonanoic acid
undecanoic acid
tridecanoic acid
pentadecanoic acid
heptadecanoic acid
nonadecanoic acid
heneicosanoic acid
tricosanoic acid
pentacosanoic acid

Preferred acids, as defined above, are those having from 7 to 15 carbon atoms in number.

(b) Penetration Enhancers

As has been stated earlier, the presence of a penetration enhancer can potentiate the benefit of the ester, by improving its delivery through the stratum corneum to its site of action in the immediate environment of the hair follicle close to the dermal papilla.

The penetration enhancer can accordingly function in a variety of ways. It can for example, improve the distribution of the ester on the skin surface or, it can increase its partition into the skin from the composition when applied topically, so aiding its passage to its site of action. Other mechanisms enhancing the benefit of the ester may also be involved.

Examples of penetration enhancers include:

2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate

Ethyl caprate
Ethyl caprylate
Butyl stearate
Benzyl salicylate
2-hydroxypropanoic acid
2-hyroxyoctanoic acid,

Further examples of penetration enhancers include:-

Dimethyl sulphoxide
N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl-2-pyrrolidone
Phosphine oxides
Sugar esters
Tetrahydrofurfural alcohol
Urea
Diethyl-m-toluamide, and
1-Dodecylazacyloheptan-2-one

Further examples of penetration enhancers include surface active agents, preferred examples of which include:

(i) Anionic surface active agents, such as metallic or alkanolamine salts of fatty acids for example sodium laurate and triethanolamine oleate;
alkyl benzene sulphonates, for example triethanolamine dodecyl benzene sulphonate;
alkyl sulphates, for example sodium lauryl sulphate;
alkyl ether sulphates, for example sodium lauryl ether sulphate [2 to 8 EO];
sulphosuccinates, for example sodium dioctyl sulphosuccinate;
monoglyceride sulphates, for example sodium glyceryl monostearate monosulphate;
isethionates, for example sodium isethionate; methyl taurides, for example Igepon T;
acylsarcosinates, for example sodium myristyl sarcosinate;
acyl peptides, for example Maypons and Lamepons;
acyl lactylates,
polyalkoxylated ether glycollates, for example trideceth-7 carboxylic acid;
phosphates, for example sodium dilauryl phosphate.

(ii) Cationic surface active agents, such as amine salts, for example sapamin hydrochloride;
quartenary ammonium salts, for example Quaternium 5, Quaternium 31 and Quaternium 18;

(iii) i) Amphoteric suface active agents, such as imidazol compounds, for example Miranol;
N-alkyl amino acids, such as sodium cocaminopropionate and asparagine derivatives;
betaines, for example cocoamidopropylbetaine

(iv) Nonionic surface active agents, such as fatty acid alkanolamides, for example oleic ethanolamide;
esters of polyalcohols, for example Span;
polyglycerol esters, for example that esterified with C12-18 fatty acids and one or several OH groups;
polyalkoxylated derivatives, for example polyoxy:polyoxyethylene stearate, and octylphenoxy polyethoxyethanol (TRITON X-100);
ethers, for example polyoxyethylene lauryl ether;
ester ethers, for example Tween;
amine oxides, for example coconut and dodecyl dimethyl amine oxides.

Mixtures of two or more of the above surface active agents can be employed in the composition according to the invention.

(c) cationic polymers chosen from:

Guar Hydroxypropyltrimonium chloride
Quaternium-19
Quaternium-23
Quaternium-40
Quaternium-57
Poly(dipropyldiallylammonium chloride)
Poly(methyl-β-propaniodiallylammonium chloride)
Poly(diallylpiperidinium chloride)
Poly(vinyl pyridinium chloride)
Quaternised poly (vinyl alcohol)
Quaternised poly
(dimethylaminoethylmethacrylate); and mixtures thereof

The amount of activity enhancer, when employed in accordance with the invention, will normally be from 0.1 to 50%, preferably from 0.5 to 25% and most preferably from 0.5 to 10% by weight of the composition.

Other hair growth promoter adjuncts

The composition according to the invention can also contain adjuncts other than those already mentioned, depending on the form of the intended product. It is, for example, possible to include antiseptics, preservatives, antioxidants, emulsifiers and colouring agents, which can improve the stability and consumer appeal of the composition.

The composition according to the invention can also be employed as a vehicle for a wide variety of cosmetically or pharmaceutically active ingredients, particularly ingredients which have some beneficial effect other than the promotion of hair growth when applied to the skin.

Process

The invention also provides a process for the preparation of a composition suitable for topical application to mammalian skin or hair which comprises mixing a ester of citric acid, as herein defined, with a suitable vehicle to provide a composition according to the invention, in which the ester forms from 0.0001 to 99% by weight of the composition.

Product Form and Container

The compositions of the invention are preferably formulated as liquids, for example as a lotion, shampoo, milk or cream for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example sticks, creams or gels, for use in conjunction with a suitable applicator or simply a tube, bottle or lidded jar, or as a liquid-impregnated fabric, such as a tissue wipe, provided penetration of the ester into the scalp is not inhibited by absorbtion of the ester into solid carriers.

The invention accordingly also provides a closed container containing a composition as herein defined.

Use of the ester of citric acid for Inducing, Maintaining or Increasing Hair Growth

The invention also provides for the use of an ester of citric acid, as herein defined, for topical application to mammalian skin or hair for inducing, maintaining or increasing hair growth.

The compositions according to the invention are primarily intended for topical application to the scalp of the human subject, particularly where the head is already bald or balding, in order to promote the regrowth of terminal hair. The compositions can also be applied prophilactically to the hair and hence the scalp to reduce or prevent the onset of baldness.

The amount of the composition and the frequency of application to the hair and/or scalp can vary widely, depending on personal needs, but it is suggested as an example that topical application of from 0.1 to 5g daily containing from 0.00001 to 1g of a selected ester over the period of at least six months will in most cases result in an improvement in hair growth.

EVALUATION OF EFFICACY OF ESTERS OF CITRIC ACID AS HAIR GROWTH PROMOTERS USING THE RAT MODEL

The Rat Hair Growth Trial

The effect of esters of citric acid on hair growth was assessed using albino rats as an animal model. The rats were chosen from as few litters as possible and were each approximately 27-30 days of age at the start of the trial. Each rat was housed individually to prevent licking.

In each comparison, 6 rats were used in each group and hair growth was assessed as follows:

A small patch of normal skin (3cm x 3cm) on the upper back of each rat was selected and hairs were plucked from this area at the start of the trial (G-1 telogen). Test materials or control in a vanishing cream base or an alcoholic lotion, as appropriate, were then applied twice daily until the end of the next anagen, i.e. after a further 20 days from the start of trial (G-2 anagen). Each application of lotion amounted to about 50 mg.

At the end of G2-anagen, hair was plucked again from the same dorsal area, and this was used for measurement of:

i) weight of hair per unit area of skin,
ii) length of hair, and
iii) diameter of hair measured microscopically.

This procedure was employed to compare the effect of topical application to the rat model of a known hair growth promoter, namely minoxidil and a test material as used in accordance with the invention, namely tri-n-butyl citrate.

In this experiment minoxidil was employed at a concentration of 2% by weight in 65% v/v aqueous ethanol and tri-n-butyl citrate was employed at a concentration of 5% by weight in a vanishing cream base.

The measurements obtained from plucked hair as detailed above are summarised in Table 1 below:

TABLE 1

| Effect of 5% by weight tri-n-butyl citrate (TBC) in a vanishing cream base, or 2% by weight minoxidil in 65% v/v aqueous ethanol, on hair growth at G-2 Anagen of albino rats (6 in each group) | | | |
|---|---|---|---|
| Hair Parameter | Group I* (Control) | Group II ('TBC') | Group III** (Minoxidil) |
| WT/AREA (mg/cm2) | 13.50 ± 0.66 | 17.24 ± 0.97 | 16.27 ± 0.97 |
| | | (II = ~ III > I) | |
| LENGTH (mm) | 8.06 ± 0.19 | 10.74 ± 1.16 | 8.85 ± 0.42 |
| | | (II > III = ~ I) | |
| DIAMETER (pa) | | | |
| MEDIAN: | 104.4 ± 5 | 109.2 ± 6 | 129.0 ± 6 |
| | | (III > II = ~ I) | |
| MAXIMUM: | 112 ± 5 | 117 ± 6 | 141 ± 7 |
| | | (III > II = ~ I) | |

* control = vanish cream base
** 5 out of 6 rats survived.

Conclusions

The results shown in Table 1 above, showed that:

1. With application of control, TBC or minoxidil products, the following differences in response were apparent at the end of the G-2 anagen growth cycle:

(a) so far as weight of plucked hair per unit area is concerned, the values of TBC and minoxidil-treated skin were similar, but both showed a marked increase over the control for this parameter.

(b) so far as length of hair is concerned, the values for minoxidil and the control were similar, while that for TBC-treated skin showed a marked increase over both control and minoxidil for this parameter.

(c) so far as hair diameter is concerned, the values for TBC and control-treated skin were similar, while that for minoxidil showed a marked increase over both control and TBC for this parameter.

It can be concluded from these results that there was a significant increase in weight of hair produced from rats treated topically with TBC or minoxidil, as compared with the control containing neither of these materials. In the case TBC treatment, this was due more to a substantial increase in hair length rather than a marginal increase in hair diameter, whereas in the case of minoxidil, the reverse was apparent. Accordingly, even though the specific physiological response of TBC as compared with that of minoxidil was apparently different, overall, TBC was shown in terms of weight of hair produced per unit area to be at least as effective as minoxidil in promoting hair growth.

This confirms that TBC is useful as a hair growth promoter and is preferred in any case to minoxidil, in view of the total lack of toxicity problems that limit the use of minoxidil as a topically applied hair growth promoter.

Examples

The invention is illustrated by the following examples.

Example 1

This Example illustrates a lotion according to the invention which is suitable for topical application to the scalp in order to promote hair growth.

The lotion has the following formulation:

| | % w/w |
|---|---|
| 2-acetyl-tri-n-butyl citrate | 5 |
| ethanol | 60 |
| water | 35 |

Example 2

This Example illustrates a hair tonic lotion which is suitable for application to hair or scalp.

The hair tonic has the following formulation:

| | % w/w |
|---|---|
| triethyl citrate | 4 |
| ethanol | 60 |
| water | 46 |
| perfume | q.s. |

Example 3

This Example also illustrates a lotion which is suitable for topical application to the scalp.

The lotion has the following formulation:

| | % w/w |
|---|---|
| 2-O-ethyl tri-n-butyl citrate | 6 |
| propan-2-ol | 10 |
| ethanol | 82 |
| perfume | q.s. |

Example 4

This Example also illustrates a hair tonic which is suitable for application to hair or scalp. The hair tonic has the following formulation:

| | % w/w |
|---|---|
| tri-n-butyl citrate | 5 |
| ethanol | 40 |
| water | 55 |
| perfume | q.s. |

Examples 5 to 8

The following formulations represent lotions which can be used topically in the treatment of bald or balding male or female heads.

| | % w/w | | | |
|---|---|---|---|---|
| | 5 | 6 | 7 | 8 |
| Hydroxyethyl cellulose | 0.4 | - | 0.4 | - |
| Absolute ethanol | 25 | 25 | 25 | 25 |
| Propane-1,2-diol | - | - | 38.4 | 38.4 |
| Butane-1,3-diol | 38.4 | 38.8 | - | - |
| Paramethyl benzoate | 0.2 | 0.2 | 0.2 | 0.2 |
| trihexyl citrate | 5 | - | - | - |
| trioctyl citrate | - | 4 | - | - |
| tridodecyl citrate | - | - | 3 | - |
| trihexadecyl citrate | - | - | - | 6 |
| Perfume | 1 | 1 | 1 | 1 |
| Water | to 100 | 100 | 100 | 100 |

Examples 9 to 12

The following formulations represent creams which can be used in the treatment of baldness.

| | % w/w | | | |
|---|---|---|---|---|
| | 9 | 10 | 11 | 12 |
| Cetyl alcohol polyoxyethylene (10) | 4 | 4 | 4 | 4 |
| Cetyl alcohol | 4 | 4 | 4 | 4 |
| Mineral oil | 4 | 2 | - | - |
| Paraffin wax | - | 2 | 4 | - |
| Partial glyceride of palmitic and stearic acids | - | - | - | 4 |
| triphenyl citrate | 2 | - | - | - |
| tri-n-butyl citrate | - | 3 | - | - |
| 2-acetyl trimethyl citrate | | - | 5 | - |
| 2-acetyl triethyl citrate | - | - | - | 6 |
| Triethanolamine | 0.75 | 0.75 | 0.75 | 0.75 |
| Butane-1,3-diol | 3 | 3 | 3 | 3 |
| Xanthan gum | 0.3 | 0.3 | 0.3 | 0.3 |
| Preservative | 0.4 | 0.4 | 0.4 | 0.4 |
| Perfume | q.s. | q.s. | q.s. | q.s. |
| Water | to 100 | 100 | 100 | 100 |

Example 13

This Example illustrates a water-in-oil high internal phase emulsion containing an ester according to the invention. The emulsion consisted of 10% by volume oily phase and 90% by weight aqueous phase.
The oily phase and the aqueous phase had the following constitution:

| | % w/w |
|---|---|
| Oily phase | |
| Sorbitan monooleate | 20 |
| Quaternium-18 hectorite | 5 |
| Liquid paraffin | 75 |
| Aqueous phase | |
| acetyl tri-n-propyl citrate | 5 |
| Xanthan gum | 1 |
| Preservative | 0.3 |
| Perfume | q.s. |
| Sodium chloride (1% w/w solution) | to 100 |

The emulsion was prepared by taking 10 parts by volume of the oily phase and to it adding slowly with stirring 90 parts by volume of the aqueous phase.

The high internal phase water-in-oil emulsion so formed can be applied topically to the scalp, to improve hair growth and regrowth.

The following examples 14 to 18 illustrate shampoos for use in washing the hair and scalp, and for promoting hair growth on the scalp.

Example 14

| | % w/w |
|---|---|
| Sodium lauryl ether sulphate (2 EO) [21% AD] | 41.4 |
| Lauryl dimethylamino acetic acid betaine: [30% AD] | 4 |
| Coconut fatty acid diethanolamine | 1.5 |
| Oleyl triethoxy phosphate (BRIPHOS 03D) | 1 |
| Polyglycol-polyamine condensation resin (POLYQUART H) [50% active] | 1.5 |
| Preservative, colouring matter, salt | 0.58 |
| 2-acetyl tri-n-butyl citrate | 4 |
| Perfume | q.s. |
| Water | to 100 |

Example 15

| | % w/w |
|---|---|
| Sodium lauryl ether sulphate (2 EO) [100% AD] | 12 |
| POLYMER JR400 | 2.5 |
| BRIPHOS 03D | 2.5 |
| 2-oleoyl tri-n-butyl citrate | 4 |
| Magnesium Sulphate | 5 |
| Perfume | q.s. |
| Water | to 100 |

Example 16

The following example illustrates a lotion according to the invention which can be applied topically to the scalp to prevent hair loss and stimulate hair regrowth.

| | % w/w |
|---|---|
| tri-n-butyl citrate | 4 |
| Minoxidil | 1 |
| ethanol | 16 |
| citric acid | 1.05 |
| water | to 100 |
| pH adjusted to 4.2 with sodium hydroxide | |

Example 17

This example illustrates a shampoo which is suitable for topical application to hair in order to cleanse it, at the same time delivering an inhibitor to the scalp to enhance hair growth or regrowth.

The shampoo had the following formulation:

| | % w/w |
|---|---|
| Triethanolamine lauryl sulphate | 16.8 |
| Coconut diethanolamide | 3.0 |
| Hydroxypropylmethylcellulose (1) | 0.25 |
| Corn syrup (80% solids) (2) | 20.5 |
| Dimethylpolysiloxane (3) | 1.0 |
| Cationic cellulose (4) | 0.5 |
| Ethyl alcohol (SDA 40) | 9.0 |
| Vinyl carboxy polymer (5) | 0.75 |
| tri-n-butyl citrate | 10 |
| Perfume, colour, preservative | q.s. |
| Water | to 100 |
| Acid or base to pH: | 6.5 |

1 - Methocel E4M (Dow Chemical)
2 - 42 Dextrose equivalent (Staley 1300)
3 - 60,000 centistokes (Viscasil, GEC)
4 - Polymer JR 400
5 - Carbopol 941 (BF Goodrich)

Examples 18 to 19

The following formulations represent lotions which can be used topically in the treatment of bald or balding male or female heads.

| | | % w/w |
|---|---|---|
| | 18 | 19 |
| Hydroxyethyl cellulose | 0.4 | - |
| Absolute ethanol | 25 | 25 |
| Propane-1,2-diol | - | - |
| Butane-1,3-diol | 38.4 | 38.8 |
| Paramethyl benzoate | 0.2 | 0.2 |
| tri-n-butyl citrate | 5 | - |
| trioctyl citrate | - | 1 |
| Perfume | 1 | 1 |
| Water | to 100 | 100 |

## Claims

1. The use of a preserved composition which comprises:
an effective amount of from 1% to 99% by weight of an ester of citric acid having the structure (1):

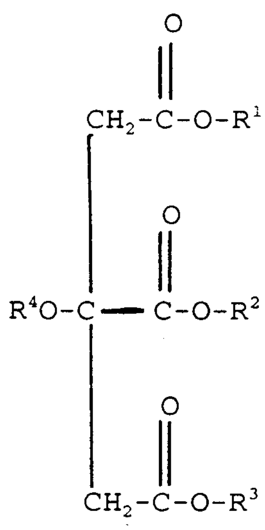

(1)

where
$R^1$, $R^2$ and $R^3$ each independently represent branched or unbranched alkyl, alkenyl, aryl, alklaryl or arylalkyl group each said group having from 1 to 18 carbon atoms,
$R^4$ represents -H, or a branched or unbranched saturated or unsaturated acyl, alkyl, aryl, alkylaryl or alrylalkyl group having from 1 to 18 carbon atoms,
in the presence of a cosmetically acceptable vehicle for the citric acid ester and in the absence of solid absorbent for the ester;
for inducing, maintaining or increasing hair growth following topical application to human scalp or hair,
said effective amount of said ester being sufficient to increase hair growth in the rat, when said composition is applied

topically thereto over a period of no more than three months, by at least 10% more than that obtainable using a control composition from which the said ester has been omitted, in accordance with the Rat Hair Growth Test.

2. The use according to claim 1, in which the ester of citric acid is tri-n-butyl citrate.

3. The use according to claim 1 or 2, in which the amount of the ester of citric acid is from 1 to 10% by weight of the composition.

4. The use according to any preceding claim which further comprises an activity enhancer.

5. The use according to claim 4, in which the vehicle acts as an activity enhancer.

6. The use according to claims 4 or 5, in which the activity enhancer is another hair growth stimulant.

7. The use according to claim 6, in which the other hair growth stimulant is minoxidil.

8. The use according to claim 4, in which the activity enhancer is a penetration enhancer.

9. The use according to claim 8, in which the penetration enhancer is a surface active agent.

10. The use according to claim 4, in which the activity enhancer is a cationic polymer.

11. The use according to any preceding claim in which the composition has a pH value of from 2 to <7.

12. The use of an ester of citric acid having the structure (1), as defined in claim 1, as an agent for converting vellus hair to grow as terminal hair.

13. The use of an ester of citric acid having the structure (1), as defined in claim 1, as an agent for increasing the rate of growth of terminal hair.

14. The use of an ester of citric acid having the structure (1), as defined in claim 1, as an agent for promoting, maintaining or enhancing hair growth.

**Patentansprüche**

1. Verwendung eines konservierten Mittels, umfassend:

eine wirksame Menge von 1 bis 99 Gew.-% eines Esters von Zitronensäure der Struktur (1):

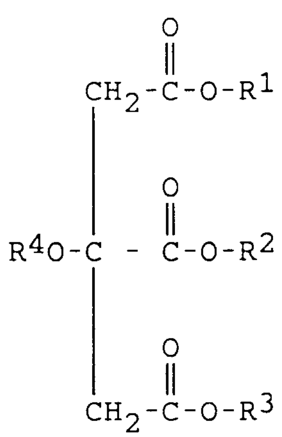

(1)

worin $R^1$, $R^2$ und $R^3$ jeweils unabhängig voneinander eine verzweigte oder nichtverzweigte Alkyl-, Alkenyl-, Aryl-, Alkylaryl- oder Arylalkylgruppe bedeutet, wobei jede Gruppe 1 bis 18 Kohlenstoffatome aufweist,

$R^4$ -H oder eine verzweigte oder nichtverzweigte, gesättigte oder ungesättigte Acyl-, Alkyl-, Aryl-, Alkylaryl- oder Arylalkylgruppe mit 1 bis 18 Kohlenstoffatomen bedeutet,

in Gegenwart eines kosmetisch verträglichen Vehiculums für den Ester von Zitronensäure und in Abwesenheit von festen Absorptionsmitteln für den Ester;

zur Anregung, Aufrechterhaltung oder Verstärkung von Haarwuchs nach örtlicher Anwendung auf die Kopfhaut oder das Haar beim Menschen, wobei die wirksame Menge des Esters ausreichend ist, um Haarwuchs bei der Ratte, wenn das Mittel örtlich darauf über einen Zeitraum von nicht mehr als 3 Monaten aufgetragen wird, um mindestens 10 % mehr als erhältlich unter Verwendung eines Kontrollmittels, bei dem der Ester fortgelassen wurde, zu erhöhen im Einklang mit dem Rattenhaarwuchstest.

**2.** Verwendung nach Anspruch 1, wobei der Ester von Zitronensäure Zitronensäure-tri-n-butylester ist.

**3.** Verwendung nach Anspruch 1 oder 2, wobei die Menge an Ester der Zitronensäure 1 bis 10 Gew.-% des Mittels ist.

**4.** Verwendung nach einem vorangehenden Anspruch, die außerdem einen Aktivitätsverstärker umfaßt.

**5.** Verwendung nach Anspruch 4, wobei das Vehiculum als Aktivitätsverstärker wirkt.

**6.** Verwendung nach den Ansprüchen 4 oder 5, wobei der Aktivitätsverstärker ein anderes Haarwuchs stimulierendes Mittel ist.

**7.** Verwendung nach Anspruch 6, wobei das andere Haarwuchs stimulierende Mittel Minoxidil ist.

**8.** Verwendung nach Anspruch 4, wobei der Aktivitätsverstärker ein Eindringverstärker ist.

**9.** Verwendung nach Anspruch 8, wobei der Eindringverstärker ein Tensid ist.

**10.** Verwendung nach Anspruch 4, wobei der Aktivitätsverstärker ein kationisches Polymer ist.

**11.** Verwendung nach einem vorangehenden Anspruch, wobei das Mittel einen PH-Wert von 2 bis <7 aufweist.

**12.** Verwendung eines Esters von Zitronensäure mit der Struktur (1) nach Anspruch 1 als Mittel zur Umwandlung von Flaumhaar zum Aufwachsen als Endhaar.

**13.** Verwendung eines Esters von Zitronensäure mit der Struktur (1) nach Anspruch 1 als Mittel zur Erhöhung der Wachstumsgeschwindigkeit des Endhaares.

**14.** Verwendung eines Esters von Zitronensäure mit der Struktur (1) nach Anspruch 1 als Mittel zur Anregung, Aufrechterhaltung oder Verstärkung des Haarwuchses.

## Revendications

**1.** L'utilisation d'une composition qui se conserve et comprenant :

une quantité efficace comprise entre 1 % et 99 % en masse d'un ester de l'acide citrique présentant la

structure (1) :

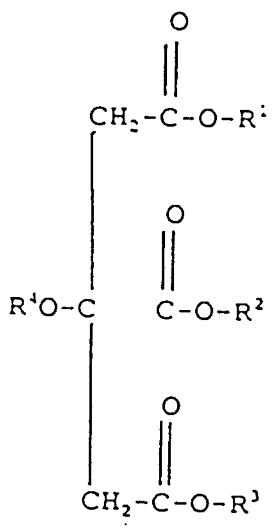

(1)

où

$R^1$, $R^2$ et $R^3$ représentent chacun indépendamment un groupe alkyle, alkényle, aryle, alkylaryle ou arylalkyle linéaire ou ramifié, chacun desdits groupes présentant 1 à 18 atomes de carbone,

$R^4$ représente -H, ou un (groupe acyle, alkyle, aryle, alkylaryle ou arylalkyle saturé ou insaturé, linéaire ou ramifié, présentant 1 à 18 atomes de carbone,

en présence d'un véhicule pour l'ester de l'acide citrique qui soit acceptable d'un point de vue cosmétique et en absence d'un absorbant solide de l'ester;

pour induire, préserver ou favoriser la croissance des cheveux suite à une application locale sur le cuir chevelu ou les cheveux des hommes, ladite quantité efficace du dit ester étant suffisante pour augmenter la croissance des poils du rat, lorsque ladite composition est appliquée localement dessus pendant une période n'excédant pas trois mois, d'au moins 10 % de plus que celle pouvant être obtenue en utilisant une composition témoin dans laquelle ledit ester a été omis, en accord avec le Test de Croissance des Poils du Rat.

2. L'utilisation selon la Revendication 1, dans laquelle l'ester de l'acide citrique est du citrate de tri-n-butyle.

3. L'utilisation selon la Revendication 1 ou 2, dans laquelle la quantité de l'ester de l'acide citrique est comprise entre 1 et 10 % en masse de la composition.

4. L'utilisation selon l'une quelconque des Revendications précédentes, qui comprend en outre un renforçateur d'activité.

5. L'utilisation selon la Revendication 4, dans laquelle le véhicule agit comme renforçateur d'activité.

6. L'utilisation selon les Revendications 4 ou 5, dans laquelle le renforçateur d'activité est un autre stimulant de la croissance des cheveux.

7. L'utilisation selon la Revendication 6, dans laquelle l'autre stimulant de la croissance des cheveux est du minoxydile.

8. L'utilisation selon la Revendication 4, dans laquelle le renforçateur d'activité est un activateur de pénétration.

9. L'utilisation selon la Revendication 8, dans laquelle l'activateur de pénétration est un agent tensioactif.

10. L'utilisation selon la Revendication 4, dans laquelle le renforçateur d'activité est un polymère cationique.

11. L'utilisation selon l'une quelconque des Revendications précédentes, dans laquelle la composition présente une valeur de pH comprise entre 2 et <7.

12. L'utilisation d'un ester de l'acide citrique présentant la structure (1), telle que définie dans la Revendication 1, en tant qu'agent permettant de convertir les cheveux vellus pour croître en cheveux terminaux.

13. L'utilisation d'un ester de l'acide citrique présentant la structure (1), telle que définie dans la Revendication 1, en tant qu'agent permettant d'augmenter le taux de croissance des cheveux terminaux.

14. L'utilisation d'un ester de l'acide citrique présentant la structure (1), telle que définie dans la Revendication 1, en tant qu'agent permettant de favoriser, de conserver ou d'améliorer la croissance des cheveux.